(19) Europäisches Patentamt
European Patent Office
Office européen des brevets

(11) Numéro de publication : **0 106 776**
**B1**

(12) # FASCICULE DE BREVET EUROPÉEN

(45) Date de publication du fascicule du brevet : **22.10.86**

(51) Int. Cl.[4] : **C 07 D263/28**, C 07 D413/12, A 61 K 31/42, A 61 K 31/435

(21) Numéro de dépôt : **83450025.8**

(22) Date de dépôt : **30.09.83**

(54) **Nouvelles amino-2 aminométhyl-5 oxazolines-2.**

(30) Priorité : **05.10.82 FR 8216782**
**19.05.83 FR 8308477**

(43) Date de publication de la demande :
**25.04.84 Bulletin 84/17**

(45) Mention de la délivrance du brevet :
**22.10.86 Bulletin 86/43**

(84) Etats contractants désignés :
**BE CH DE GB IT LI LU NL**

(56) Documents cités :
**FR-A- 2 202 701**
**FR-M- 2 448**
**US-A- 3 637 726**
**JOURNAL OF MEDICINAL CHEMISTRY, vol. 16, no. 5, mai 1973, WASHINGTON D.C. (US) E.R. FREITER et al.: "Some 2-amino-5-substituted oxazolines and intermediates as potential anorectants", pages 510-512**

(73) Titulaire : **SOCIETE CORTIAL S.A.**
**7 rue de l'Armorique**
**F-75015 Paris (FR)**

(72) Inventeur : **Creuzet, Marie-Hélène**
**Résidence Jardin de Gambetta T3**
**F-33000 Bordeaux (FR)**
Inventeur : **Feniou, Claude**
**5 rue du Général Guillomat**
**F-33600 Pessac (FR)**
Inventeur : **Jarry, Christian**
**11 rue des Mésanges**
**F-33370 Artigues près Bordeaux (FR)**
Inventeur : **Prat, Gisèle**
**Hameau de Noailles Villa 18**
**F-33400 Talence (FR)**
Inventeur : **Pontagnier, Henri**
**21 rue Edouard Vaillant**
**F-33600 Pessac (FR)**

(74) Mandataire : **Tajan, Marie-Thérèse**
**LABORATOIRES SARGET Avenue du Président JF Kennedy**
**F-33701 Mérignac (FR)**

**EP 0 106 776 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne de nouvelles amino-2 aminométhyl-5 oxazolines-2, leur méthode de préparation ainsi que leur application thérapeutique.

Les produits de la présente invention ont pour formule générale

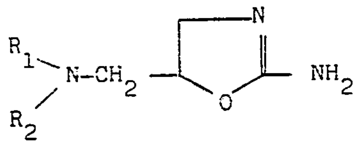

(I)

avec $R_1$, $R_2$ identiques ou différents représentant un groupement alkyl choisi dans le groupe $CH_3$, $C_2H_5$, $C_3H_7$, $C_4H_9$ ou un groupement benzyl ou phényl ou bien $R_1$ et $R_2$ formant avec l'atome d'azote auquel ils sont rattachés un hétérocycle appartenant au groupe pipéridine, pyrrolidine, morpholine, tétrahydroisoquinoléine ou bien pipéridine ou pipérazine substituée par R avec R = alkyl inférieur de $C_1$ à $C_4$, allyl, benzyl, pyridyl, phényl substitué ou non par un ou plusieurs substituants appartenant au groupe formé par chloro, fluoro, bromo, trifluorométhyl, méthyl, méthoxy, hydroxy.

On connaît déjà des amino-2 oxazolines-2. Ainsi l'amino-2 phényl-5 oxazoline-2

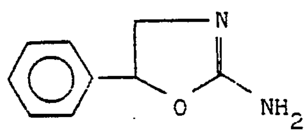

a été brevetée par la société MacNeil Laboratories Incorporated en France sous le numéro 2448 M pour ses propriétés de stimulation du système nerveux central et son activité anorexigène. L'amino-2 (dichloro-3,4 phénoxyméthyl)-5 oxazoline-2 a été testée par A. H. Abdallah et coll. pour son activité cardiovasculaire et anorexigène (Toxicol. appl. Pharmacol., 1973, 26, 513-22 ; 1973, 25, 344-53) et a été brevetée par la Société Dow Chemical aux Etats-Unis sous le n° 3637726 le 9 avril 1970 pour son activité antimicrobienne.

Les produits de la présente invention se distinguent des amino-2 oxazolines-2 déjà connues par la présence en position 5 du cycle d'une substitution aminométhyl. Cette substitution aminométhyl-5 apparaît toutefois dans le brevet FR 12 202 701 dans une série de dérivés de structure aryl-4 aminométhyl-5 amino-2 oxazole de formule générale

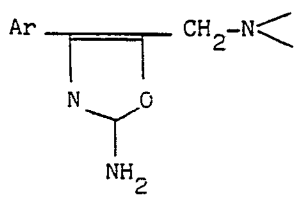

représentant une forme tautomère de produits appartenant à la formule plus générale

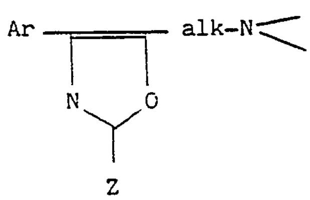

avec Z : O ou NH, alk = $(CH_2)_{1\ à\ 3}$ et Ar représentant un groupe aromatique.

Les produits de la présente invention présentent des propriétés pharmacologiques permettant leur application en thérapeutique notamment cardiovasculaire, psychotrope, antiinflammatoire, antiallergique, antihistaminique, antiH2.

Ces produits sont préparés par condensation, dans un solvant tel que le méthanol, du dérivé monosodé du cyanamide sur l'amino-1 époxy-2,3 propane N-substitué correspondant

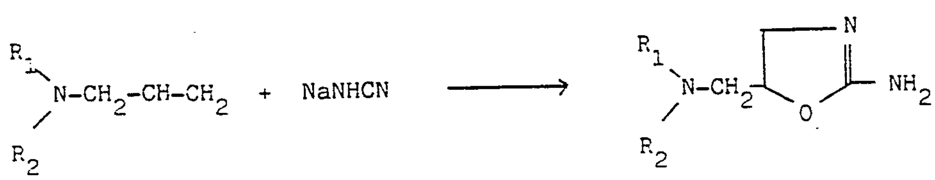

Les époxydes sont préparés de façon classique par réaction de l'amine $HNR_1R_2$ (dans laquelle $R_1$ et $R_2$ ont les significations indiquées plus haut) et de l'épichlorhydrine.

L'invention est précisée dans les exemples suivants sans toutefois que ceux-ci ne limitent sa portée.

## Exemple 1

Préparation de l'amino-2 N,N-diéthylaminométhyl-5 oxazoline-2. Formule I avec $R_1 = R_2 = C_2H_5$.

Dans un réacteur muni d'une agitation, d'un réfrigérant et d'une ampoule à brome on introduit une mole de diéthylamine. On introduit, sous agitation, goutte à goutte une mole d'épichlorhydrine en maintenant la température à 25 °C. L'agitation est poursuivie pendant 5 heures à température ambiante. On ajoute 300 $cm^3$ d'éther dans le réacteur puis 48 g de soude finement pulvérisée. L'agitation est prolongée à température ambiante pendant une nuit. On filtre et on centrifuge la phase éthérée. Celle-ci est lavée par 50 $cm^3$ d'eau. Après décantation la phase organique est séchée soigneusement sur sulfate de Na. Le solvant est éliminé sous pression réduite et le diéthylamino-1 époxy-2,3 propane est séparé par distillation. Rendement 60 %. $Eb_8$ = 44-48 °C. $Eb_{760}$ = 157 °C.

A 12,8 g du dérivé monosodé du cyanamide dissous dans 200 $cm^3$ de méthanol anhydre on ajoute goutte à goutte en agitant vigoureusement 0,2 mole de diéthylamino-1 époxy-2,3 propane ainsi préparé. Après 15 heures d'agitation à température ambiante le méthanol est évaporé et le résidu repris par 300 $cm^3$ d'éther. Le précipité est éliminé par filtration. La phase éthérée est lavée deux fois par 2 $cm^3$ d'eau puis séchée sur $Na_2SO_4$ après décantation. L'éther est évaporé sous pression réduite. La phase huileuse obtenue cristallise après lavage par de l'heptane bouillant. L'amino-2 diéthylaminométhyl-5 oxazoline-2 est ainsi obtenue avec un rendement de 68 %. Elle est purifiée par recristallisation dans l'heptane.

F = 90 °C. Masse moléculaire 171.

Spectre infrarouge : bande $\nu NH_2$ 3 360 $cm^{-1}$, $\nu C=N$ 1 680 $cm^{-1}$.

RMN du proton dans DMSOD6 (sont donnés les déplacements chimiques exprimés en ppm par rapport au TMS pris comme étalon interne) : 5,78 ppm, singulet, 2 protons ($NH_2$) ; 4,67-4,29 ppm, massif complexe, 1 proton (H-5 sur le cycle oxazoline) ; 3,78-3,05 ppm, massif complexe, 2 protons (H en 4 sur le cycle oxazoline) ; 2,71-2,31 ppm, massif complexe, 6 protons ($N—CH_2$) ; 0,93 ppm, triplet, 6 protons ($CH_3—$).

## Exemple 2

Synthèse de l'amino-2 (N-butyl N-méthylaminométhyl)-5 oxazoline-2. Formule I avec $R_1 = CH_3$, $R_2 = C_4H_9$.

Ce produit est préparé selon la méthode décrite dans l'exemple 1. L'époxyde intermédiaire présente un point d'ébullition de 44-50° sous 0,665 millibar.

Le produit final est purifié par recristallisation dans l'hexane.

F = 86 °C. Masse moléculaire 185.

Spectre infrarouge : $\nu NH_2$ 3 280 $cm^{-1}$, $\nu C=N$ 1 680 $cm^{-1}$.

Spectre RMN dans le DMSOD6 : 5,8 ppm, singulet, 2 protons ($NH_2$) ;
4,71-4,33 ppm, massif complexe, 1 proton (H en 5 du cycle oxazoline) ;
3,78-3 ppm, massif complexe, 2 protons (H en 4 du cycle oxazoline) ;
2,62-2,1 ppm, massif complexe, 7 protons

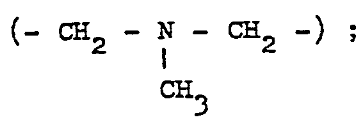

1,55-1,05 ppm, massif complexe, 4 protons ($C—CH_2—CH_2—C$) ; 1-0,71 ppm, massif complexe, 3 protons ($CH_3—C$).

## Exemple 3

Synthèse de l'amino-2 pyrrolidinométhyl-5 oxazoline-2. Formule I avec $R_1$, $R_2 = —CH_2—CH_2—CH_2—CH_2—$

Ce produit est préparé selon la méthode décrite dans l'exemple 1 à la seule différence que la réaction d'addition de la pyrrolidine et de l'épichlorhydrine est effectuée en présence d'éther. L'époxyde intermédiaire présente un point d'ébullition de 73 °C sous 33,2 millibar. L'amino-2 pyrrolidinométhyl-5 oxazoline-2 est purifiée par recristallisation dans l'heptane.

F = 123 °C. Masse moléculaire 169.

Spectre infrarouge : $\nu NH_2$ = 3 300 $cm^{-1}$, $\nu C=N$ 1 685 $cm^{-1}$.

Spectre de RMN dans le DMSOD6 : 5,82 ppm, singulet, 2 protons ($NH_2$) ; 4,71-4,29 ppm, massif complexe, 1 proton (H en 5 du cycle oxazoline) ; 3,78-3,02 ppm, massif complexe 2 protons (H en 4 du cycle oxazoline) ; 2,66-2,31 ppm, massif complexe, 6 protons ($CH_2$—N) ; 1,87-1,44 ppm, massif complexe, 4 protons ($CH_2$—C).

Exemple 4

Synthèse de l'amino-2 pipéridinométhyl-5 oxazoline-2. Formule I avec $R_1$, $R_2$ = —$CH_2$—$CH_2$—$CH_2$—$CH_2$—$CH_2$—.

Ce produit est préparé selon la méthode décrite dans l'exemple 1. L'époxyde intermédiaire présente un point d'ébullition de 51 °C sous 0,665 millibar. Le produit final est purifié par recristallisation dans l'heptane.

F = 127 °C. Masse moléculaire 183.

Spectre infrarouge $\nu NH_2$ 3 350 $cm^{-1}$, $\nu C = N$ 1 680 $cm^{-1}$.

Spectre de RMN dans le DMSOD6 : 5,8 ppm, singulet, 2 protons ($NH_2$) ; 4,77-4,35 ppm, massif complexe, 1 proton (H en 5 du cycle oxazoline) ; 3,77-2,95 ppm, massif complexe, 2 protons (H en 4 du cycle oxazoline) ; 2,58-2,16 ppm, massif complexe, 6 protons ($CH_2$—N) ; 1,64-1,2 ppm, massif complexe, 6 protons ($CH_2$—C).

Exemple 5

Synthèse de l'amino-2 (méthyl-4 pipéridinométhyl)-5 oxazoline-2. Formule I avec

$$R_1,\ R_2 = -CH_2-CH_2-\underset{CH_3}{\underset{|}{C}H}-CH_2-CH_2-$$

Ce produit est synthétisé selon la méthode décrite dans l'exemple 1. L'époxyde intermédiaire présente un point d'ébullition de 55 °C sous 0,665 millibar. Le produit final est recristallisé dans l'heptane.

F = 130 °C. Masse moléculaire 197.

Spectre infrarouge : $\nu NH_2$ 3 260 $cm^{-1}$, $\nu C = N$ 1 690 $cm^{-1}$.

Spectre de RMN dans le DMSOD6 : 5,82 ppm, singulet, 2 protons ($NH_2$) ; 4,78-4,33 ppm, massif complexe, 1 proton (H en 5 du cycle oxazoline) ; 3,78-3 ppm, massif complexe, 2 protons (H en 4 du cycle oxazoline) ; 3-0,9 ppm, massif complexe, 11 protons ($CH_2$—N + H du cycle pipéridine) ; 0,87 ppm, doublet, 3 protons ($CH_3$).

Exemple 6

Synthèse de l'amino-2 (éthyl-2 pipéridinométhyl)-5 oxazoline-2. Formule I avec

$$R_1,\ R_2 = -\underset{C_2H_5}{\underset{|}{C}H}-CH_2-CH_2-CH_2-CH_2-$$

Ce produit est synthétisé selon la méthode décrite dans l'exemple 1. L'époxyde intermédiaire présente un point d'ébullition de 83 °C sous 0,665 millibar. Le produit final est recristallisé dans l'heptane.

F = 114 °C. Masse moléculaire 211.

Spectre infrarouge : $\nu NH_2$ 3 350 $cm^{-1}$, $\nu C = N$ 1 680 $cm^{-1}$.

Spectre de RMN dans le DMSO6 : 5,82 ppm, singulet, 2 protons ($NH_2$) ; 4,75-4,33 ppm, massif complexe, 1 proton (H en 5 du cycle oxazoline) ; 3,86-3,05 ppm, massif complexe, 2 protons (H en 4 du cycle oxazoline) ; 3-2,05 ppm, massif complexe, 5 protons ($CH_2$—N + CH—N) ; 1,77-1,11 ppm, massif complexe, 8 protons ($CH_2$—C) ; 0,8 ppm, triplet, 3 protons ($CH_3$).

Exemple 7

Synthèse de l'amino-2 morpholinométhyl-5 oxazoline-2. Formule I avec $R_1R_2$ = —$CH_2$—$CH_2$—O—$CH_2$—$CH_2$—

Ce produit est synthétisé selon la méthode décrite dans l'exemple 1. L'époxyde intermédiaire présente un point d'ébullition de 67 °C sous 0,665 millibar. Le produit final est purifié par recristallisation dans $CCl_4$.

F = 152 °C. Masse moléculaire 185.

Spectre infrarouge : $\nu NH_2$ 3 340 $cm^{-1}$, $\nu C = N$ 1 680 $cm^{-1}$.

Spectre de RMN dans le DMSOD6 5,84 ppm, singulet, 2 protons ($NH_2$) ;

4,82-4,38 ppm, massif complexe, 1 proton (H en 5 du cycle oxazoline) ;

3,77-3 ppm, massif complexe, 2 protons (H en 4 du cycle oxazoline) ;

3,55 ppm, triplet, 4 protons ($CH_2$—O) ; 2,58-2,29 ppm, massif complexe, 6 protons ($CH_2$—N).

Exemple 8

Synthèse de l'amino-2 (N-méthyl N-phénylaminométhyl)-5 oxazoline-2. Formule I avec $R_1 = CH_3$, $R_2 = C_6H_5$.

Ce produit est synthétisé selon la méthode décrite dans l'exemple 1 à la différence près que tout au long de la réaction entre la N-méthylaniline et l'épichlorhydrine, le mélange réactionnel est chauffé à 50 °C. L'époxyde intermédiaire présente un point d'ébullition de 105 °C sous 0,665 millibar. Le produit final se présente sous forme d'une huile indistillable. Masse moléculaire 205.

Spectre infrarouge : $\nu NH_2$ 3 340 $cm^{-1}$, $\nu C = N$ 1 680 $cm^{-1}$.

Spectre de RMN dans le DMSOD6 : 7,4-6,4 ppm, massif complexe, 5 protons (H du cycle aromatique) ; 5,65 ppm, massif complexe, 2 protons ($NH_2$) ;

4,89-4,44 ppm, 4-3,05 ppm, massif complexe, 4 protons ($CH_2$) ; 3 ppm, triplet, 3 protons ($CH_3$).

Exemple 9

Synthèse de l'amino-2 (N-benzyl N-méthylaminométhyl)-5 oxazoline-2. Formule I avec $R_1 = CH_2C_6H_5$, $R_2 = CH_3$.

Ce produit est synthétisé selon la méthode décrite dans l'exemple 1. Le produit final est recristallisé dans l'heptane.

F = 98 °C. Masse moléculaire 219.

Spectre infrarouge : $\nu NH_2$ 3 490 $cm^{-1}$, $\nu C = N$ 1 680 $cm^{-1}$.

Spectre RMN dans $CDCl_3$ : 7,22 ppm, singulet, 5 protons (H aromatiques) ;

4,84 ppm, singulet, 2 protons ($NH_2$) ;

4,82-4,48 ppm, massif complexe, 1 proton (H en 5 du cycle oxazoline) ;

3,88-3,18 ppm, massif complexe, 2 protons (H en 4 du cycle oxazoline) ;

3,53 ppm, singulet, 2 protons ($CH_2$ benzylique) ;

2,82-2,17 ppm, massif complexe, 2 protons ($CH_2$ en 5 sur le cycle oxazoline) ;

2,29 ppm, singulet, 3 protons ($NCH_3$).

Exemple 10

Synthèse de l'amino-2 (méthyl-4 pipérazinyl) méthyl-5 oxazoline-2. Formule I avec

$R_1NR_2$ = 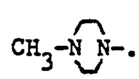

A une solution de 0,25 mole de N-méthylpipérazine dans 200 $cm^3$ d'éther, on ajoute goutte à goutte 0,25 mole (23,13 g) d'épichlorhydrine. Le mélangeur réactionnel est maintenu à une température inférieure à 35 °C pendant l'addition ; il est ensuite chauffé au bain-marie pendant deux heures à la température d'ébullition de l'éther. 10 g (0,25 mole) de soude finement pulvérisée sont ajoutés. Le précipité est éliminé par filtration.

La phase éthérée est lavée par deux fois deux $cm^3$ d'eau puis séchée sur $Na_2SO_4$. Le solvant est éliminé et le méthylpipérazinyl-1 époxy-2,3 propane est séparé par distillation.

Point d'ébullition sous 1,33 mb = 100 °C.

La pureté de ce produit est contrôlée en infrarouge par disparition de la bande NH de l'amine de départ et apparition d'une bande C—O—C à 930 $cm^{-1}$. A 0,1 mole (15,6 g) de N-méthyl pipérazinyl-1 époxy-2,3 propane dissoute dans 150 $cm^3$ de méthanol on ajoute 0,1 mole (6,4 g) de dérivé monosodé du cyanamide en solution dans 100 $cm^3$ de méthanol. Après 15 heures d'agitation à température ambiante le méthanol est évaporé et le résidu repris dans 200 $cm^3$ d'éther éthylique. Le précipité est éliminé par filtration. La phase éthérée est lavée rapidement avec 5 $cm^3$ d'eau puis séchée sur $Na_2SO_4$. L'éther est évaporé sous presssion réduite. L'amino-2 N-méthyl pipérazinylméthyl-5 oxazoline-2 est recueillie sous forme d'huile qui prend en masse puis est recristallisée dans $CCl_4$.

F = 130 °C. Rendement 61 %. Masse moléculaire 198. Microanalyse : calculé C 9,09 %, H 54,54 %, N

28,28 % ; trouvé C 9,13 %, H 54,51 %, N 28,19 %. Spectre IR : bandes $\nu NH_2$ 3 300 et 3 180 $cm^{-1}$, bande $\nu C = N$ 1 680 $cm^{-1}$.

RMN du proton dans $CDCl_3$

2,1-3,0 ppm, 13 protons, massif complexe,

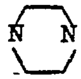 $—CH_2 + CH_3$ à 2,3 ppm ;

3,1-4,1 ppm, 2 protons, massif complexe, $CH_2$ oxazolinique ; 4,4-5,0 ppm, 1 proton, massif complexe, CHO ; 5,4 ppm, 2 protons, pic large, $NH_2$, échangeable avec $D_2O$.

Exemple 11

Synthèse de l'amino-2 (phényl-4 pipérazinyl) méthyl-5 oxazoline-2. Formule I avec

$R_1NR_2 = C_6H_5-$ 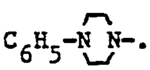 .

Ce produit est préparé selon la méthode décrite dans l'exemple 10. L'époxyde intermédiaire est obtenu sous forme cristallisée. F = 89 °C ; (solvant de recristallisation heptane).

Le produit final est purifié par recristallisation dans l'heptane. F = 174 °C. Rendement 51 %. Masse moléculaire 260. Microanalyse : calculé C 7,69 %, H 64,62 %, N 21,54 % ; trouvé C 7,60 %, H 64,40 %, N 21,34 %. Spectre IR : bandes $\nu NH_2$ 3 330 et 3 180 $cm^{-1}$, bande $\nu C = N$ 1 670 $cm^{-1}$. RMN du proton dans $CDCl_3$

2,3-4,1 ppm, 12 protons, massif complexe, $6CH_2$ ; 4,5-5,0 ppm, 3 protons, massif complexe, CHO + $NH_2$, échangeable avec $D_2O$ ; 6,7-7,5 ppm, 5 protons, massif complexe, protons aromatiques.

Exemple 12

Synthèse de l'amino-2 (benzyl-4 pipéridinyl) méthyl-5 oxazoline-2. Formule I avec

$R_1NR_2 = C_6H_5CH_2$— 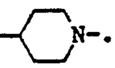 .

Ce produit est synthétisé selon la méthode décrite dans l'exemple 10. Son rendement d'obtention est de 46 %. Il est purifié par recristallisation dans l'heptane.

F = 114 °C. Masse moléculaire 273. Spectre IR : bandes $\nu NH_2$ 3 320 et 3 160 $cm^{-1}$, bande $\nu C = N$ 1 685 $cm^{-1}$.

RMN du proton dans $CDCl_3$

1,1-4,1 ppm, 15 protons, massif complexe, 7 $CH_2$ + CH pipéridinique ;

4,4-5,0 ppm, 1 proton, massif complexe, CHO ; 5,1 ppm, 2 protons, pic large, $NH_2$, échangeable avec $D_2O$ ; 6,9-7,4 ppm, 5 protons, massif complexe, protons aromatiques.

Exemple 13

Synthèse de l'amino-2 (benzyl-4 pipérazinyl) méthyl-5 oxazoline-2. Formule I avec

$R_1NR_2 = C_6H_5CH_2-$ 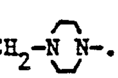 .

Ce produit est synthétisé selon la méthode décrite dans l'exemple 10. Son rendement d'obtention est de 48 %. Il est purifié par recristallisation dans l'heptane.

F = 96 °C. Masse moléculaire 274. Microanalyse calculé C 8,03 %, H 65,69 %, N 20,44 % ; trouvé C 8,06 %, H 64,93 %, N 20,34 %.

Spectre IR : bandes $\nu NH_2$ 3 310 et 3 160 $cm^{-1}$, bande $\nu C = N$ 1 680 $cm^{-1}$.

RMN du proton dans $CDCl_3$

2,2-3,0 ppm, 10 protons, massif complexe,

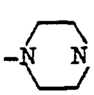 $-CH_2$ ;

3,1-4,1 ppm, 4 protons, massif complexe, $CH_2$ benzylique + $CH_2$ oxazolinique ; 4,4-5,0 ppm, 3 protons, massif complexe, CHO + $NH_2$ échangeable avec $D_2O$ ; 7,2-7,4 ppm, 8 protons, massif complexe, protons aromatiques.

Exemple 14

Synthèse de l'amino-2 (allyl-4 pipérazinyl) méthyl-5 oxazoline-2. Formule I avec

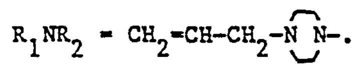

Ce produit est synthétisé selon la méthode décrite dans l'exemple 10. L'époxyde intermédiaire présente un point d'ébullition de 67 °C sous 1 mb. Le produit final obtenu avec un rendement de 43 % est purifié par recristallisation dans l'heptane.

F = 99 °C. Masse moléculaire 224. Microanalyse : calculé C 8,93 %, H 58,93 %, N 25 % ; trouvé C 8,93 %, H 58,01 %, N 24,99 %.

Spectre IR : bandes $\nu NH_2$ 3 200 et 3 160 $cm^{-1}$, bande $\nu C = N$ 1 690 $cm^{-1}$.

RMN du proton dans $CDCl_3$

2,2-4,1 ppm, 14 protons, massif complexe,

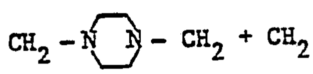

oxazolinique ; 4,4-6,3 ppm, 6 protons, massif complexe, $CH_2 = CH—$ + CHO + $NH_2$ à 4,9 ppm échangeable avec $D_2O$.

Les propriétés pharmacologiques des produits de la présente invention sont exposées ci-après.

La toxicité a été déterminée chez la souris pour diverses voies d'administration. Le tableau n° 1 donne les pourcentages de mortalité en fonction des doses administrées.

(Voir Tableau I page 8)

Le produit de l'exemple 1 présente après administration par voie i.v. chez la souris une DL 50 de 128 (116-142 mg/kg).

L'activité antihistaminique anti$H_2$ a été déterminée in vitro sur oreillette droite de cobaye battant spontanément. Ainsi les produits des exemples 1, 2, 6, 13 inhibent à plus de 50 % à la concentration de 100 microg/ml l'effet chronotrope induit par 5 microg/ml d'histamine, sans présenter d'effet chronotrope intrinsèque. Le produit de l'exemple 14 présente cette activité à la concentration de 50 microg/ml.

L'activité inotrope positive a été déterminée in vitro sur oreillette gauche de cobaye stimulée électriquement. Le produit de l'exemple 1 entraîne à 100 microg/ml 100 % d'augmentation de la force contractile de l'oreillette.

L'activité antiallergique a été déterminée chez le rat dans le test de l'anaphylaxie passive cutanée. Les animaux sensibilisés par une injection intradermique d'IgE reçoivent immédiatement après l'administration du produit à tester par voie intraveineuse une injection d'ovalbumine et de bleu d'Evans. Les produits des exemples 3 et 4 entraînent à 10 mg/kg respectivement 53 et 60 % d'inhibition de la tache de bleu d'Evans. Le produit de l'exemple 11 entraîne 60 % d'inhibition quand il est administré à 100 mg/kg *per os*.

L'activité diurétique a été déterminée chez des rats soumis à un régime hydrique et appréciée par le rapport du sodium excrété chez les animaux traités au sodium excrété chez les animaux témoins. Les produits des exemples 3 et 7 multiplient l'excrétion du sodium respectivement par 4,2 et 4,9 à une dose de 20 mg/kg *per os*.

L'activité hypocholestérolémiante a été déterminée chez des souris soumises à un régime riche en cholestérol et acide cholique pendant 7 jours. Après administration *per os* du produit de l'exemple 6 à la dose de 400 mg/kg le 6ᵉ et le 7ᵉ jour, on note une diminution de la cholestérolémie de 21 %. Cette chute de la cholestérolémie est accompagnée d'une diminution de 20 % de la fraction HDI-VLDL, le rapport HPL/cholestérol étant égal à 1,01.

Les activités psychotropes antidépressives sont mises en évidence dans les tests suivants. Les produits des exemples 11 et 12 administrés à la dose orale de 100 mg/kg potentialisent la toxicité de la yohimbine. Le produit de l'exemple 11 administré à 50 mg/kg *per os* inhibe le ptosis induit par la réserpine.

L'activité anti-inflammatoire a été mise en évidence dans le test de l'œdème à la carragénine chez le rat. Le produit de l'exemple 11 administré *per os* à la dose de 100 mg/kg entraîne à l'échéance de 4 heures 35 % d'inhibition de l'œdème à la carragénine. A la dose de 200 mg/kg administré *per os* ce produit est très peu ulcérigène.

L'activité antihistaminique anti$H_1$ a été déterminée *in vitro* sur iléon de cobaye. Les produits des

| Produits des exemples | Pourcentage de mortalité aux doses de voie orale 300 mg/kg | 200 mg/kg | voie intrapéritonéale 200 mg/kg | 100 mg/kg |
|---|---|---|---|---|
| 1 | 0 | | 0 | |
| 2 | 0 | | 100 | 0 |
| 3 | 0 | | 100 | 0 |
| 4 | 33 | 0 | 100 | 0 |
| 5 | 0 | | 100 | 0 |
| 6 | 0 | | 0 | |
| 7 | 100 | | | |
| 10 | 0 | | 0 | |
| 11 | 0 | | 0 | |
| 12 | 100 | 0(1) | 100 | 0 |
| 13 | 0 | | 100 | 0 |
| 14 | 0 | | 0 | |

(1) : 100 mg/kg

exemples 11 et 13 inhibent à plus de 80 % les contractions de l'iléon de cobaye induites par l'histamine quand on les utilise respectivement aux concentrations de 25 et 100 microg/ml.

*In vitro* le produit de l'exemple 11 entraîne à la concentration de 25 microg/ml, 80 % d'inhibition des contractions induites par la sérotonine sur l'iléon de cobaye.

L'activité antiulcérigène a été mise en évidence dans le test de l'ulcère de stress. Le produit de l'exemple 11 administré à la dose de 25 mg/kg *per os* entraîne 88 % d'inhibition de l'ulcère de stress du rat.

Dans le modèle du rat spontanément hypertendu, le produit de l'exemple 13 administré à la dose de 100 mg/kg par voie orale entraîne une chute de la pression systolique de respectivement 14 et 15 % aux échéances de deux et quatre heures. Le produit de l'exemple 14 administré à la dose de 100 mg/kg dans les mêmes conditions entraîne une diminution de 21 % de la pression systolique à l'échéance de six heures.

Compte tenu de leurs activités pharmacoliques jointes à une toxicité relativement faible les produits faisant l'objet de la présente invention peuvent être employés en thérapeutique humaine ou animale.

Ainsi, associés aux excipients appropriés, ils pourront être utilisés dans le traitement des états dépressifs, des états inflammatoires et œdémateux, de la crise d'asthme ou de tout état allergique, de l'hypertension artérielle, des hypersécrétions gastriques, des ulcères gastroduodénaux. Leur activité inotrope positive permet de les employer dans le traitement des défaillances cardiaques aiguës ou chroniques. Leurs propriétés diurétiques permettent de les employer dans le traitement des œdèmes et rétentions hydrosodés. Leurs propriétés hypolipidémiantes permettent de les employer dans les hypercholestérolémies et les hypertriglycéridémies résistant au régime.

Ils seront administrés par exemple par voie orale sous forme de dragées, comprimés, sirop, ampoules, par voie rectale sous forme de suppositoires, par voie intramusculaire ou intraveineuse ou par voie topique sous forme de pommades ou gels. Les doses administrées varieront selon l'indication et le sujet de 1 à 100 mg/j en 2 à 6 prises pour la voie orale, de 1 à 100 mg/j en 1 ou 2 prises pour la voie rectale, de 0,5 à 50 mg par injection pour les voies parentérales. Ils pourront également être employés en inhalation par exemple sous forme de nébulisats.

## Revendications

1. Produits de formule générale

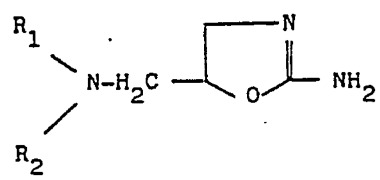

avec $R_1$, $R_2$ identiques ou différents représentant un groupement alkyl choisi dans le groupe $CH_3$, $C_2H_5$, $C_3H_7$, $C_4H_9$ ou un groupement benzyl ou phényl ou bien $R_1$ et $R_2$ formant avec l'atome d'azote auquel ils sont rattachés un hétérocycle appartenant au groupe pipéridine, pyrrolidine, morpholine, tétrahydroisoquinoléïne ou bien pipéridine ou pipérazine substituée par R avec R = alkyl inférieur de C1 à C4, allyl, benzyl, pyridyl, phényl substitué ou non par un ou plusieurs substituants appartenant au groupe formé par chloro, fluoro, bromo, trifluorométhyl, méthyl, méthoxy, hydroxy.

2. Méthode de préparation des produits selon la revendication 1 caractérisée en ce que l'on condense dans un solvant tel que le méthanol, le dérivé monosodé du cyanamide sur un amino-1 époxy-2,3 propane n-substitué de formule

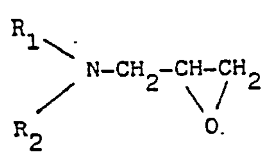

avec $R_1$, $R_2$ identiques ou différents représentant un groupement alkyl choisi dans le groupe $CH_3$, $C_2H_5$, $C_3H_7$, $C_4H_9$ ou un groupement benzyl ou phényl ou bien $R_1$ et $R_2$ formant avec l'atome d'azote auquel ils sont rattachés un hétérocycle appartenant au groupe pipéridine, pyrrolidine, morpholine, tétrahydroisoquinoléine ou bien pipéridine ou pipérazine substituée par R avec R = alkyl inférieur de $C_1$ à $C_4$, allyl, benzyl, pyridyl, phényl substitué ou non par un ou plusieurs substituants appartenant au groupe formé par chloro, fluoro, bromo. trifluorométhyl, méthyl, méthoxy, hydroxy.

3. Médicament caractérisé en ce que le principe actif est constitué par au moins un produit selon la revendication 1.

4. Médicament utile en thérapeutique cardiovasculaire caractérisé en ce que le principe actif est constitué par au moins un produit selon la revendication 1.

5. Médicament utile en allergologie caractérisé en ce que le principe actif est constitué par au moins un produit selon la revendication 1.

6. Médicament utile dans la thérapeutique des ulcères gastroduodénaux caractérisé en ce que le principe actif est constitué par au moins un produit selon la revendication 1.

7. Médicament utile en thérapeutique psychotrope caractérisé en ce que le principe actif est constitué par au moins un produit selon la revendication 1.

8. Composition pharmaceutique ou vétérinaire caractérisée en ce qu'elle contient à titre de principe actif au moins un produit selon la revendication 1.

## Claims

1. Products with the general formula

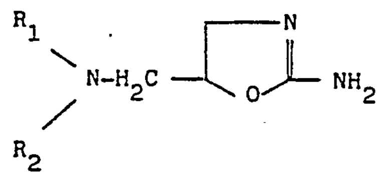

with $R_1$, $R_2$ identical or different being an alkyl grouping selected in the group $CH_3$, $C_2C_5$, $C_3H_7$, $C_4H_9$, or a benzyl or phenyl grouping, or else $R_1$ and $R_2$ forming together with that nitrogen atom they are linked to, a heterocycle from the group piperidine, pyrrolidine, morpholine, tetrahydro-isoquinoline, or else R-substituted piperidine or piperazine, with R = lower alkyl from $C_1$ to $C_4$, allyl, benzyl, pyridyl, phenyl substituted or not with one or a plurality of substituents from the group comprised of chloro, fluoro, bromo, trifluoromethyl, methyl, methoxy, hydroxy.

2. Method for preparing products as defined in claim 1, which comprises condensing in a solvent such as methanol, the monosodio derivative from cyanamide, on a N-substituted amino-1 epoxy-2,3 propane with as formula

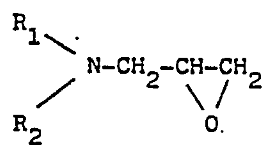

with $R_1$, $R_2$ identical or different being an alkyl grouping selected in the group $CH_3$, $C_2H_5$, $C_3H_7$, $C_4H_9$, or a benzyl or phenyl grouping, or else $R_1$ and $R_2$ forming together with that nitrogen atom they are linked to, a heterocycle from the group piperidine, pyrrolidine, morpholine, tetrahydro-isoquinoline, or else R-substituted piperidine or piperazine, with R = lower alkyl from $C_1$ to $C_4$, allyl, benzyl, pyridyl, phenyl substituted or not with one or a plurality of substituents from the group comprised of chloro, fluoro, bromo, trifluoromethyl, methyl, methoxy, hydroxy.

3. Medicament, in which the active constituent is comprised of at least one product as defined in claim 1.

4. Medicament useful for cardiovascular therapy, in which the active constituent is comprised of at least one product as defined in claim 1.

5. Medicament useful for allergy therapy, in which the active constituent is comprised of a least one product as defined in claim 1.

6. Medicament useful for gastro-duodenal ulcer therapy, in which the active constituent is comprised of at lest one product as defined in claim 1.

7. Medicament useful for psychotrope therapy, in which the active constituent is comprised of at least one product as defined in claim 1.

8. Pharmaceutic or veterinary compound, which contains as active constituent, at least one product as defined in claim 1.

## Patentansprüche

1. Verbindungen der allgemeinen Formel

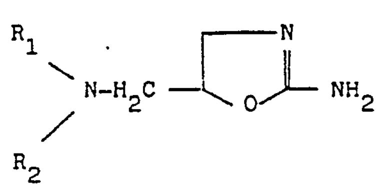

in der $R_1$ und $R_2$, die gleich oder verschieden sein können, aus der von $CH_3$, $C_2H_5$, $C_3H_7$ und $C_4H_9$ gebildeten Gruppe ausgewählte Alkylgruppen oder Benzyl- oder Phenylgruppen bedeuten oder zusammen mit dem Stickstoffatom, an das sie gebunden sind, einen Heterocyclus aus der von Piperidin, Pyrrolidin, Morpholin, Tetrahydroisochinolin oder durch R substituiertem Piperidin oder Piperazin gebildeten Gruppe bilden, wobei R niedriges Alkyl mit C1 bis C4, Allyl, Benzyl, Pyridyl, mit einem Substituenten oder mehreren aus der Gruppe Chlor, Fluor, Brom, Trifluormethyl, Methyl, Methoxy und Hydroxy substituiertes Phenyl oder unsubstituiertes Phenyl bedeutet.

2. Verfahren zur Herstellung der Verbindungen nach Anspruch 1, dadurch gekennzeichnet, daß man in einem Lösemittel wie Methanol das Mononatriumderivat des Cyanamids mit einem N-substituierten Amino-1-epoxy-2,3-propan der Formel

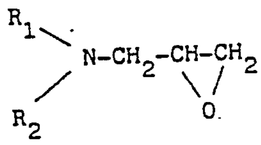

kondensiert, in der $R_1$ und $R_2$, die gleich oder verschieden sein können, aus der von $CH_3$, $C_2H_5$, $C_3H_7$ und $C_4H_9$ gebildeten Gruppe ausgewählte Alkylgruppen oder Benzyl- oder Phenylgruppen bedeuten oder zusammen mit dem Stickstoffatom, an das sie gebunden sind, einen Heterocyclus aus der von Piperidin, Pyrrolidin, Morpholin, Tetrahydroisochinolin oder durch R substituiertem Piperidin oder Piperazin gebildeten Gruppe bilden, wobei R niedriges Alkyl mit C1 bis C4, Allyl, Benzyl, Pyridyl, mit einem Substituenten oder mehreren aus der Gruppe Chlor, Fluor, Brom, Trifluormethyl, Methyl, Methoxy und Hydroxy substituiertes Phenyl oder unsubstituiertes Phenyl bedeutet.

3. Arzneimittel, dadurch gekennzeichnet, daß es als Wirkstoff mindestens eine der Verbindungen nach Anspruch 1 enthält.

4. Arzneimittel zur Verwendung in der cardiovaskulären Therapie, dadurch gekennzeichnet, daß es als Wirkstoff mindestens eine der Verbindungen nach Anspruch 1 enthält.

5. Arzneimittel zur Verwendung in der Allergologie, dadurch gekennzeichnet, daß es als Wirkstoff mindestens eine der Verbindungen nach Anspruch 1 enthält.

6. Arzneimittel zur Verwendung in der Therapie von gastroduodenalen Geschwüren, dadurch gekennzeichnet, daß es als Wirkstoff mindestens eine der Verbindungen nach Anspruch 1 enthält.

7. Arzneimittel zur Verwendung in der psychotropen Therapie, dadurch gekennzeichnet, daß es als Wirkstoff mindestens eine der Verbindungen nach Anspruch 1 enthält.

8. Pharmazeutische oder veterinärmedizinische Zusammensetzung, dadurch gekennzeichnet, daß sie als Wirkstoff mindestens eine der Verbindungen nach Anspruch 1 enthält.